(19) Europäisches Patentamt
European Patent Office
Office européen des brevets

(11) Veröffentlichungsnummer: **0 570 724 B1**

(12) **EUROPÄISCHE PATENTSCHRIFT**

(45) Veröffentlichungstag der Patentschrift: **08.02.95**

(21) Anmeldenummer: **93106705.2**

(22) Anmeldetag: **26.04.93**

(51) Int. Cl.[6]: **C07D 413/04**, C07F 9/06, A61K 31/41, A61K 31/445, A61K 33/42

(54) **Salze von 3-(cis-2,6-Dimethylpiperidino)-sydnonimin.**

(30) Priorität: **16.05.92 DE 4216310**

(43) Veröffentlichungstag der Anmeldung:
**24.11.93 Patentblatt 93/47**

(45) Bekanntmachung des Hinweises auf die Patenterteilung:
**08.02.95 Patentblatt 95/06**

(84) Benannte Vertragsstaaten:
**AT BE CH DE ES FR GB GR IT LI NL SE**

(56) Entgegenhaltungen:
**EP-A- 0 327 808**

**JOURNAL OF CARDIOVASCULAR PHARMACOLOGY Bd. 18, Nr. 4, 1991, Seiten 522 - 527BOHN H ETAL. 'CAS 936, a novel sydnonimine with direct vasodilating and nitric oxide-donating properties: effects on isolated blood vessels'**

**CIRCULATORY SHOCK Bd. 38, Nr. 3, 1992, Seiten 209 - 216 CAREY C ET AL.'Antishock and endothelial protective actions of a NO donor in mesentric ischemia and reperfusion'**

(73) Patentinhaber: **CASSELLA Aktiengesellschaft**
**Hanauer Landstrasse 526**
**D-60386 Frankfurt (DE)**

(72) Erfinder: **Schönafinger, Karl, Dr.**
**Holunderweg 8**
**W-8755 Alzenau (DE)**
Erfinder: **Kujath, Eckard, Dr.**
**Florscheidstrasse 31**
**W-6457 Maintal 1 (DE)**
Erfinder: **Voegele, Dieter, Dr.**
**Kurt-Schumacher-Ring**
**W-6454 Bruchköbel (DE)**

(74) Vertreter: **Muley, Ralf, Dr.**
**Cassella AG,**
**Patentabteilung,**
**Hanauer Landstrasse 526**
**D-60386 Frankfurt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft 3-(cis-2,6-Dimethylpiperidino)-sydnoniminium-dihydrogenphosphat und -hydrogentartrat, Verfahren zu ihrer Herstellung und ihre Verwendung.

3-(2,6-Dimethylpiperidino)-sydnonimin-hydrochlorid und seine pharmakologischen Eigenschaften sind bereits in der EP-B 327 808 beschrieben. Es hat allerdings den Nachteil, daß es beim Stehen unter dem Einfluß der Luftfeuchtigkeit Wasser aufnimmt. Die Menge des aufgenommenen Wassers ist dabei von der Temperatur und der relativen Luftfeuchtigkeit abhängig.

Das Hydrochlorid ist somit für die Standardisierung von galenischen Zubereitungen, für die vom Gesetzgeber genau definierte Wirkstoffmengen vorgeschrieben sind, kaum brauchbar. Ebenso ist die Stabilität von galenischen Zubereitungsformen, wie beispielsweise Tabletten, nicht gegeben.

Aufgabe vorliegender Erfindung ist es, den Wirkstoff in eine nicht hygroskopische Form zu bringen, die die galenischen Anforderungen erfüllt.

Diese Aufgabe wird in überraschender Weise gelöst durch ein 3-(cis-2,6-Dimethylpiperidino)-sydnonimin-Salz der allgemeinen Formel I

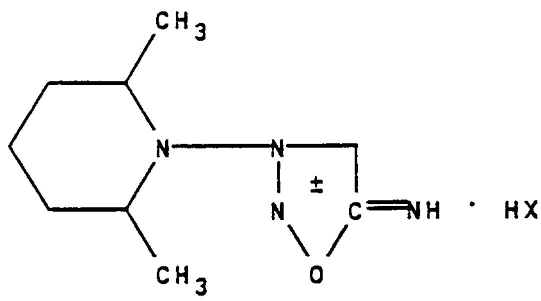

(I)

worin
X $H_2PO_4$ oder

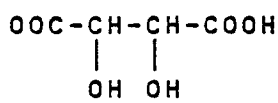

bedeutet.

Die Verbindungen der allgemeinen Formel I sind nicht hygroskopisch und besitzen daher nicht vorhergesehene Vorteile bei der Herstellung von geeigneten Arzneiformen. Bei der Herstellung von Lösungen, wie sie für i.v.-Anwendungen benötigt werden, haben sie zudem den Vorteil, daß sie in wässrigem Milieu einen schwach sauren pH-Wert einstellen, der für die Stabilität des Sydnonimins günstig und für die parenterale Anwendung akzeptabel ist.

Die Verbindungen der allgemeinen Formel I können beispielsweise dadurch hergestellt werden, daß eine Verbindung der allgemeinen Formel II

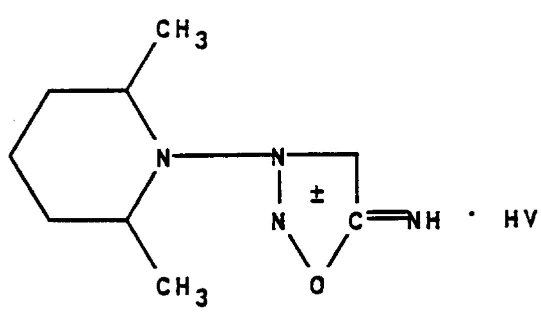

(II)

worin HV für eine beliebige Säure, wie beispielsweise Salz- oder Schwefelsäure steht, in einem Lösungsmittel mit Weinsäure beziehungsweise Phosphorsäure und einer anorganischen oder organischen Base versetzt wird.

Geeignete Lösungsmittel sind beispielsweise Wasser, Alkohole wie Methanol, Ethanol, Butanol oder i-Propanol, Wasser-Alkohol-Gemische, Acetonitril, Aceton, DMSO, DMF sowie deren Gemische mit Wasser.

Geeignete anorganische oder organische Basen sind beispielsweise Natriumhydrogencarbonat, Natriumcarbonat, Kaliumhydrogencarbonat, Kaliumcarbonat, Natriumacetat, Lithiumhydroxid, Natron- oder Kalilauge, Ammoniak, Triethylamin oder Pyridin.

Die Reihenfolge der Zugabe von Wein- bzw. Phosphorsäure und Base ist völlig unkritisch und kann beliebig verändert werden.

Der Anionenaustausch wird normalerweise bei Temperaturen von -10 bis 40 ° C, insbesondere -5 bis 30 ° C, vorzugsweise 0 bis 25 ° C, durchgeführt.

Die Verbindungen der allgemeinen Formel II können, wie in der EP-B 327 808 beispielsweise für das Hydrochlorid angegeben, hergestellt werden.

Die Verbindungen der allgemeinen Formel I können aber auch, analog zu dem in der EP-B 327 808 angegebenen Verfahren durch Cyclisierung von N-Nitroso-N-(cis-2,6-dimethylpiperidino)-aminoacetonitril der Formel III

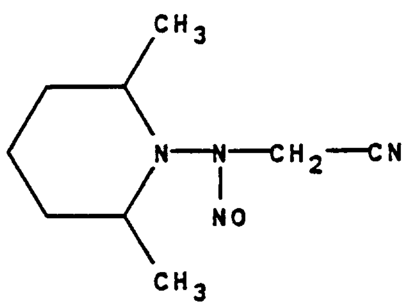

(III)

mit Phosphorsäure beziehungsweise mit Weinsäure, hergestellt werden. Das Verhältnis Verbindung der Formel III: Säure beträgt dabei bevorzugt 1 : 1 bis 1 : 10.

Geeignete Lösungsmittel für diese Umsetzung sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methylethylether, Di-n-propyl-ether, Di-isopropyl-ether, Methyl-n-butylether, Methyl-tert-butylether, Ethyl-propylether, Di-butyl-ether, Tetrahydrofuran, 1,4-Dioxan, 1,2-Dimethoxyethan, Bis-$\beta$-methoxyethylether; Oligoethylen-glykol-dimethyl-ether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl- oder -isobutylester, Essigsäure-methyl-, -ethyl-, -propyl-, -isopropyl-, -butyl-, -isobutyl- oder -sec-butyl-, -amyl-, -iso-amyl-, -hexyl-, -cyclohexyl- oder -benzyl-ester, Propionsäure-methyl-, -ethyl- oder -butyl-ester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig- und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol, Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugsweise Essigsäureethylester-Methanol.

Die Cyclisierung wird normalerweise bei Temperaturen von -10 bis 40 ° C, insbesondere 0 bis 40 ° C, vorzugsweise 0 bis 20 ° C, durchgeführt.

Die Herstellung der Verbindung der Formel III kann, wie in EP-B 327 808 beschrieben, erfolgen.

Die pharmakologischen Eigenschaften der Verbindungen der allgemeinen Formel I sind von der Tatsache, daß es sich um Dihydrogenphosphate bzw. Hydrogentartrate handelt, unabhängig. Sie senken den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die Verbindungen der allgemeinen Formel I können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspenisonen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien, enthalten. Sie können auch noch andere therapeutisch wirksame Stoffe enthalten.

Derartige andere therapeutische wirksame Substanzen sind beispielsweise: $\beta$-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretika, wie z.B. Chlorothiazid; das Herz tonisierende Mittel, wie z.B. Digitalispräparate, blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der allgemeinen Formel I und pharmazeutische Präparate, welche die Verbindungen der allgemeinen Formel I als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

## Beispiele

1. 3-(cis-2,6-Dimethylpiperidino)-sydnoniminium-hydrogentartrat

Eine Lösung von 23,2 g 3-(cis-2,6-Dimethylpiperidino)-sydnonimin-Hydrochlorid in 50 ml Wasser wird im Eisbad gekühlt. Nach Zugabe von 9,2 g Natriumbicarbonat wird eine Lösung von 16,5 g L(+)-Weinsäure in 15 ml Wasser zugetropft und im Eisbad weitergerührt. Nach 2 Stunden wird der Feststoff abgesaugt und aus einem Gemisch aus gleichen Teilen Wasser und Methanol umkristallisiert. Der Feststoff wird mit Ethanol und anschließend mit Essigester gewaschen und getrocknet.

Ausbeute: 18,5 g

Fp. 170-2 °C (Zers.)

$\alpha_D^{20}$ = +12,46(c = $H_2O$/MeOH (1 : 1))

$H_2O$-Gehalt (n.Fischer) <0,1 %

2. Die Verbindung des Beispiels 1 kann auch, wie dort angegeben, aus 29,4 g 3-(cis-2,6-Dimethylpiperidino)-sydnoniminium-hydrogensulfat und 18,5 g Natriumhydrogencarbonat erhalten werden.

3. 3-(cis-2,6-Dimethylpiperidino)-sydnoniminium-dihydrogenphosphat

Zu einer eisgekühlten Lösung von 29,4 g 3-(cis-2,6-Dimethylpiperidino)-sydnoniminium-hydrogensulfat in 75 ml Wasser wird eine Mischung aus 19,8 g Diammoniumhydrogenphosphat, 30 ml Wasser und 2,2 ml 85 %iger Phosphorsäure getropft. Nach 1 Stunde Nachrühren wird der Feststoff abgesaugt.

Ausbeute: 13,7 g

Zur weiteren Reinigung kann das Produkt aus Wasser unter Zusatz von 10 % Phosphorsäure oder aus Wasser-Ethanol-, Wasser-Methanol-Gemischen umkristallisiert werden.

Fp. 147 °C (Zers.)

$H_2O$-Gehalt (n.Fischer) <0,1 %

4. Die Verbindung des Beispiels 3 kann auch dadurch erhalten werden, daß zu einer Lösung von 147 g N-Nitroso-N-(cis-2,6-dimethylpiperidino)-aminoacetonitril in 500 ml Essigester 89 g Orthophosphorsäure gegeben werden und man diese Mischung 3 Tage stehenläßt. Anschließend wird der Niederschlag abgesaugt und mit Essigester gewaschen.

Ausbeute: 96 g

Fp. 148 °C (Zers.)

$H_2O$-Gehalt (n.Fischer): 0,05 %

5. 3-(cis-2,6-Dimethylpiperidino)-sydnoniminium-hydrogentartrat

Zur Lösung von 29,4 g 3-(cis-2,6-Dimethylpiperidino)-sydnoniminium-hydrogensulfat in 100 ml Wasser wird bei 10 °C eine auf 10 °C gekühlte Lösung von 9,5 g Natriumhydroxid und 19,7 g L(+)-Weinsäure in 160 ml Wasser getropft. Nach einstündigem Nachrühren wird das ausgefallene Produkt abgesaugt, mit 1prozentiger Weinsäurelösung gewaschen und getrocknet.

Ausbeute: 27,4 g.

6. Die Verbindung des Beispiels 5 wird, wie dort angegeben, durch Zutropfen einer Lösung von 8,0 g Natriumhydroxid und 16,5 g L(+)-Weinsäure in 100 ml Wasser zu einer Lösung von 29,4 g 3-(cis-2,6-Dimethylpiperidino)-sydnoniminium-hydrogensulfat in 90 ml Wasser in einer Ausbeute von 25,2 g erhalten.

7. Die Verbindung des Beispiels 5 kann auch, wie dort angegeben, aus 88,3 g 3-(cis-2,6-Dimethylpiperidino)-sydnoniminium-hydrogensulfat in 325 ml Wasser und einer Lösung von 70,9 g Ethanolamin und 108,6 g L(+)-Weinsäure in 220 ml Wasser erhalten werden.

Ausbeute: 93,2 g.

8. Die Verbindung des Beispiels 5 wird durch Zutropfen einer Lösung von 20,2 g Triethylamin und 16,5 g Weinsäure in 30 ml Wasser zu einer eisgekühlten Lösung von 29,4 g 3-(cis-2,6-Dimethylpiperidino)-sydnoniminium-hydrogensulfat in 100 ml Wasser in einer Ausbeute von 29,3 g erhalten.

9. 3-(cis-2,6-Dimethylpiperidino)-sydnoniminium-dihydrogenphosphat

Zu einer Lösung von 29,4 g 3-(cis-2,6-Dimethylpiperidino)-sydnoniminium-hydrogensulfat in 75 ml Wasser wird eine Lösung von 15,1 g Diammoniumhydrogenphosphat und 1,6 g 85prozentiger Phosphorsäure in 23 ml Wasser getropft. Das Reaktionsgemisch wird abgekühlt, 2 Stunden im Eisbad gerührt, der Feststoff abgesaugt, mit Wasser gewaschen und getrocknet.

Ausbeute: 14,7 g.

10. Die Verbindung des Beispiels 9 wird durch Zutropfen eines Gemisches von 30,3 g Triethylamin, 31,6 g 85prozentiger Phosphorsäure und 30,0 ml Wasser zur Lösung von 29,4 g 3-(cis-2,6-Dimethylpiperidino)-sydnoniminium-hydrogensulfat in 75 ml Wasser bei 20 - 25 °C in einer Ausbeute von 20,6 g erhalten.

**Patentansprüche**

**1.** 3-(cis-2,6-Dimethylpiperidino)-sydnonimin-Salz der allgemeinen Formel I

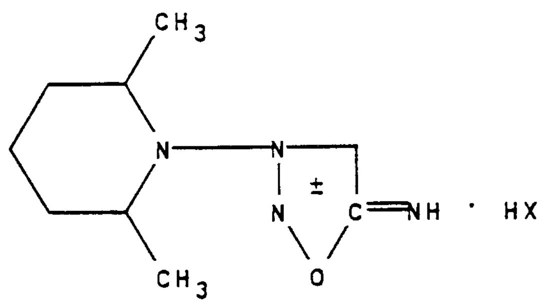

(I)

worin X $H_2PO_4$ oder

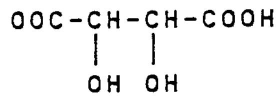

bedeutet.

**2.** Verbindung der Formel

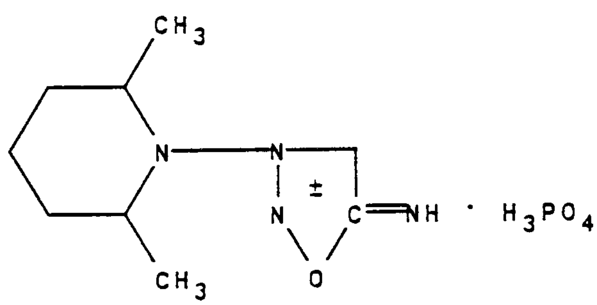

**3.** Verbindung der Formel

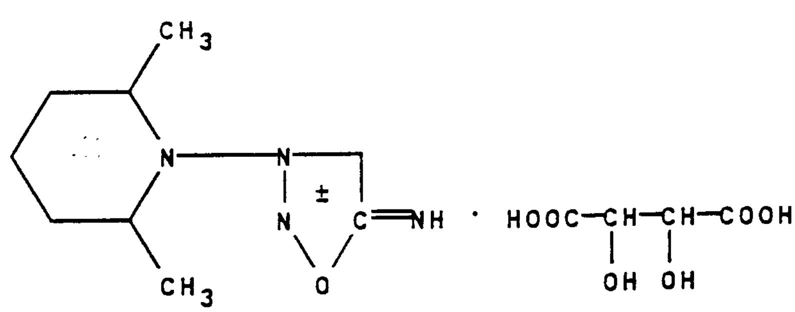

**4.** Verfahren zur Herstellung einer Verbindung der allgemeinen Formel I gemäß Anspruch 1, dadurch gekennzeichnet, daß eine Verbindung der allgemeinen Formel II

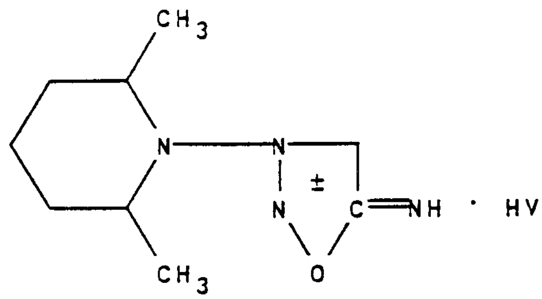

(II)

worin HV für eine beliebige Säure, wie beispielsweise Salz- oder Schwefelsäure steht, in einem Lösungsmittel mit Weinsäure beziehungsweise Phosphorsäure und der entsprechenden Menge einer anorganischen oder organischen Base versetzt wird.

5. Verfahren zur Herstellung einer Verbindung der allgemeinen Formel I gemäß Anspruch 1, dadurch gekennzeichnet, daß N-Nitroso-N-(cis-2,6-dimethylpiperidino)-aminoacetonitril der Formel III

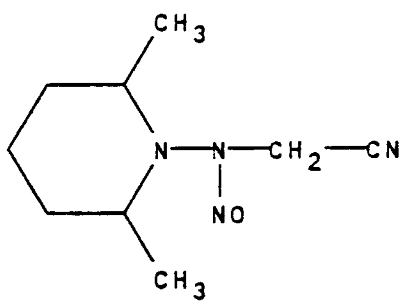

(III)

mit Phosphorsäure beziehungsweise mit Weinsäure cyclisiert wird.

6. Verwendung der Verbindungen der Ansprüche 1 bis 3 zur Bekämpfung beziehungsweise Vorbeugung von Erkrankungen des kardiovaskulären Systems.

7. Verwendung der Verbindungen der Ansprüche 1 bis 3 zur Bekämpfung beziehungsweise Vorbeugung von Angina pectoris.

8. Pharmazeutisches Präparat, dadurch gekennzeichnet, daß es eine Verbindung nach einem oder mehreren der Ansprüche 1 bis 3 als Wirkstoff zusammen mit pharmazeutisch annehmbaren Träger- und Zusatzstoffen und gegebenenfalls noch einen oder mehrere andere pharmakologische Wirkstoffe enthält.

**Claims**

1. 3-(cis-2,6-Dimethylpiperidino)sydnone imine salt of the general formula I

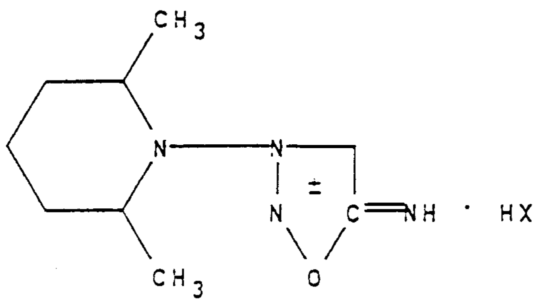

(I)

in which

X denotes $H_2PO_4$ or

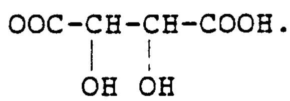

2. Compound of the formula

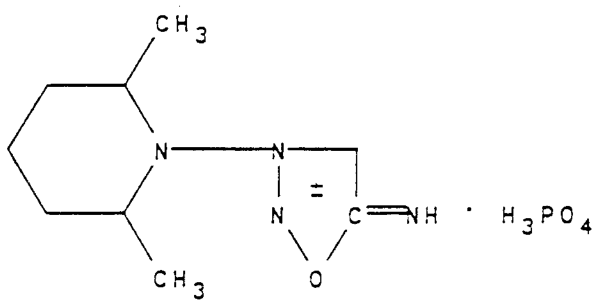

3. Compound of the formula

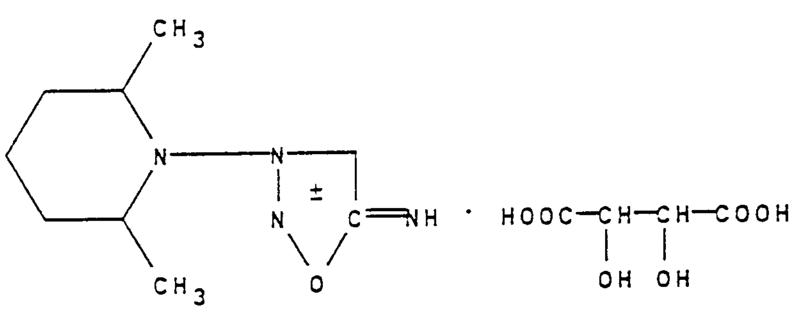

4. Process for the preparation of a compound of the general formula I according to Claim 1, characterised in that a compound of the general formula II

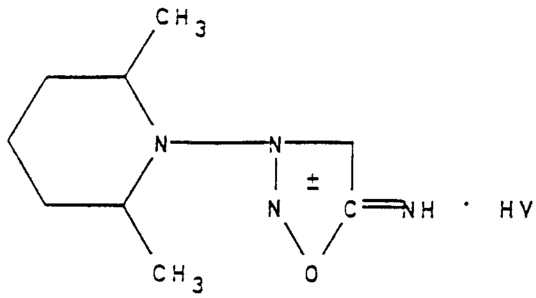

(II)

in which HV represents any desired acid, such as, for example, hydrochloric or sulphuric acid, is treated in a solvent with tartaric acid or phosphoric acid and the appropriate amount of an inorganic or organic base.

5. Process for the preparation of a compound of the general formula I according to Claim 1, characterised in that N-nitroso-N-(cis-2,6-dimethylpiperidino)aminoacetonitrile of the formula III

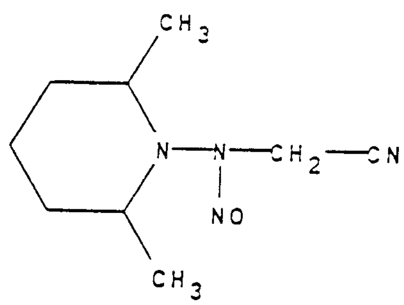

(III)

is cyclised using phosphoric acid or using tartaric acid.

6. Use of the compounds of Claims 1 to 3 for the control or prevention of disorders of the cardiovascular system.

7. Use of the compounds of Claims 1 to 3 for the control or prevention of angina pectoris.

8. Pharmaceutical preparation, characterised in that it contains a compound according to one or more of Claims 1 to 3 as active compound, together with pharmaceutically acceptable excipients and additives and, if appropriate, also one or more other pharmacological active compounds.

**Revendications**

1. Sel de 3-(cis-2,6-diméthyl-pipéridino)-sydnonimine de formule générale I :

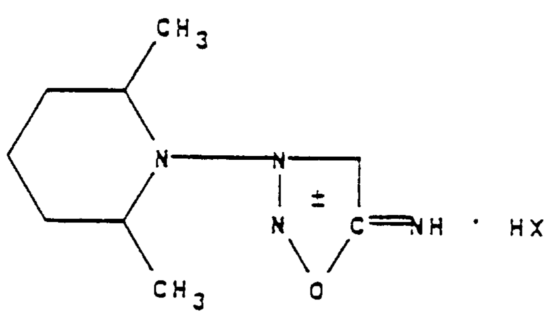

(I)

où X est $H_2PO_4$ ou

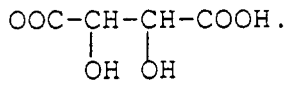

2. Composé de formule :

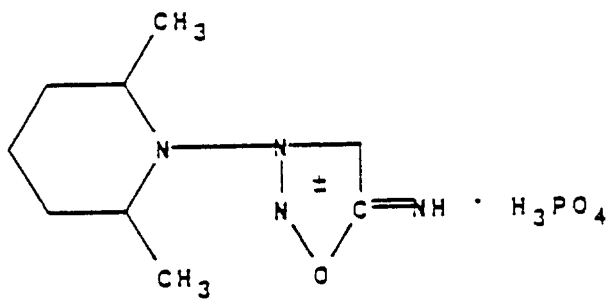

3. Composé de formule :

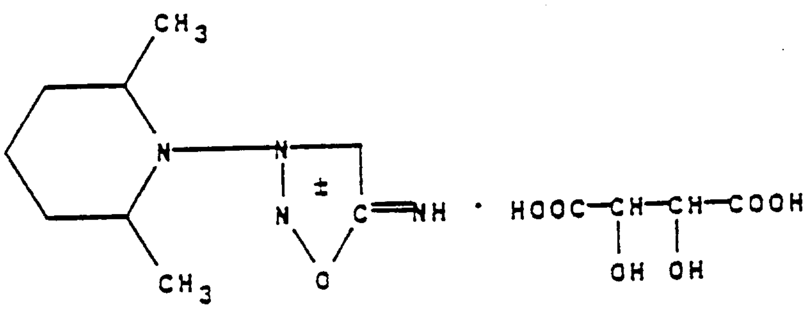

4. Procédé pour la préparation d'un composé de formule générale I selon la revendication 1, caractérisé en ce qu'on ajoute à un composé de formule générale II :

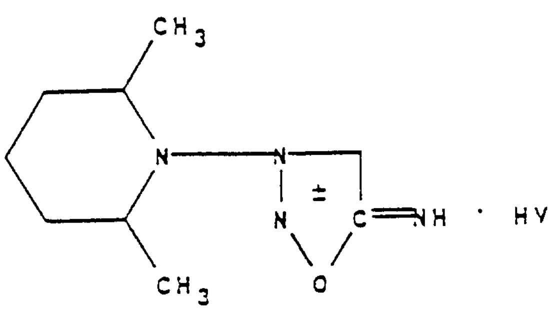

(II)

dans laquelle HV représente un acide quelconque, comme par exemple l'acide chlorhydrique ou l'acide sulfurique, de l'acide tartrique, respectivement de l'acide phosphorique, dans un solvant, et la quantité correspondante d'une base minérale ou organique.

5. Procédé pour la préparation d'un composé de formule générale I selon la revendication 1, caractérisé en ce qu'on effectue la cyclisation du N-nitroso-N-(cis-2,6-diméthylpipéridino)-aminoacétonitrile de formule III :

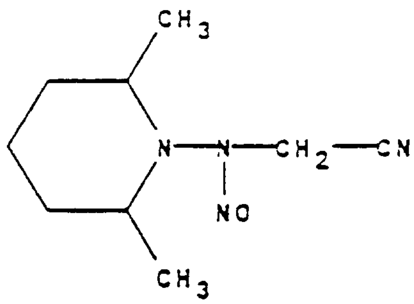

(III)

avec de l'acide phosphorique, respectivement l'acide tartrique.

**6.** Utilisation des composés selon les revendications 1 à 3 pour la lutte, respectivement la prévention, des maladies du système cardiovasculaire.

**7.** Utilisation des composés selon les revendications 1 à 3 pour la lutte, respectivement la prévention, de l'angine de poitrine.

**8.** Préparation pharmaceutique, caractérisée en ce qu'elle contient un composé selon une ou plusieurs des revendications 1 à 3 en tant que principe actif conjointement avec des adjuvants et véhicules pharmaceutiquement acceptables et, éventuellement, une ou plusieurs autres matières actives pharmacologiques.